# EUROPÄISCHE PATENTSCHRIFT

(11) **EP 4 376 757 B1**
(45) Veröffentlichungstag und Bekanntmachung des Hinweises auf die Patenterteilung: **05.03.2025**
(21) Anmeldenummer: 22758150.1
(22) Anmeldetag: 25.07.2022
(51) Int. Cl.: A61B 1/008, A61B 34/30, A61B 34/00, A61B 17/00, A61B 17/29, A61B 1/005

(54) **CHIRURGISCHES INSTRUMENT UND LENKGETRIEBE DAFÜR**
SURGICAL INSTRUMENT AND STEERING GEAR FOR SAME
INSTRUMENT CHIRURGICAL ET MÉCANISME DE DIRECTION APPROPRIÉ

(30) Priorität: 28.07.2021 DE 102021119524
(43) Veröffentlichungstag der Anmeldung: 05.06.2024
(73) Patentinhaber: Karl Storz SE & Co. KG, 78532 Tuttlingen (DE)
(72) Erfinder: SCHNEIDER, Janosz, 78532 Tuttlingen (DE); LÄNGLE, Dominik, 78532 Tuttlingen (DE); STEFAN, Jochen, 78532 Tuttlingen (DE); SPECKER, Christina, 78532 Tuttlingen (DE)
(86) Internationale Anmeldenummer: PCT/EP2022/070789
(87) Internationale Veröffentlichungsnummer: WO 2023/006663

(56) Entgegenhaltungen:
- WO-A1-2021/127686
- WO-A2-2020/218920
- DE-A1- 102019 121 092
- US-A- 5 454 827
- US-A1- 2005 090 809
- US-A1- 2008 221 391

## Beschreibung

Die Erfindung betrifft ein chirurgisches Instrument und ein Lenkgetriebe dafür.

Aus dem Stand der Technik sind chirurgische Instrumente bekannt, die manuell oder von einem Roboter geführt werden können und die Werkzeuge aufweisen, deren Werkzeugspitze mittels mehrerer ineinandergreifender Schwenkglieder verschwenkt werden kann. Diese Schwenkglieder sind mit einer Vielzahl Lenkdrähte oder -seile verbunden, um eine feinfühlige Steuerung der Werkzeugspitze zu erreichen. Mit vielen dünnen Lenkdrähten gegenüber wenigen dickeren Lenkdrähten kann eine gleichmäßigere Kraftverteilung in alle Abwinkelungsrichtungen erzielt werden.

Ein gattungsgemäßes chirurgisches Instrument ist bspw. aus der US 5 454 827 bekannt, bei dem die distalseitigen Schwenkglieder über vier Lenkdrähte mit einer proximalseitig angeordneten räumlich verstellbaren Taumelscheibe so gekoppelt sind, dass eine Bewegung der räumlich verstellbaren Taumelscheibe eine entsprechende relative Bewegung der distalseitigen Schwenkglieder und somit ein Verschwenken der Werkzeugspitze verursacht, wobei das Bewegen der räumlich verstellbaren Taumelscheibe manuell über eine Art Joystick erfolgt, der direkt mit dieser gekoppelt ist.

Die Ausbildung des Antriebs für die Lenkdrähte mit der räumlich verstellbaren Taumelscheibe, an der alle vier Lenkdrähte gelagert sind, hat den Vorteil, dass dies eine räumlich kompakte Bauweise ermöglicht und nur ein Bauteil bewegt werden muss, um alle Lenkdrähte ansprechen zu können.

Nachteilig an dieser bekannten Konstruktion ist die Verwendung einer nur geringen Anzahl von Lenkdrähten, nämlich von nur vier Lenkdrähten, und ferner die ausschließlich manuelle Betätigbarkeit der als Antrieb für die Lenkdrähte dienenden räumlich verstellbaren Scheibe, wodurch eine feinfühlige und reproduzierbare Verstellung der distalseitigen Schwenkglieder kaum möglich ist.

In der US 7 699 855 ist ein chirurgisches Instrument offenbart, das eine Schnittstelle aufweist, um das Instrument mit einem Roboter-Arm verbinden zu können. Dabei sind alle Antriebe, die das Instrument steuern, in dem Roboter-Arm angeordnet. Die Übertragung der Drehwinkel von Antrieben zum Instrument erfolgt über Kupplungsscheiben in einer gemeinsamen Trenn-Ebene.

Die WO 2014 / 004 242 beschreibt ebenfalls eine solche Schnittstelle, wobei die Antriebe in dem Roboter-Arm verbaut sind.

Die vorstehende Gestaltung ist verbunden mit einem komplexen Aufbau und einer indirekten und spielbehafteten Ansteuerung. Die Antriebe sind nicht in dem chirurgischen Instrument direkt angeordnet, woraus ein nicht lineares Übertragungsverhalten bei der Ansteuerung der Taumelscheibe resultiert, das nur schlecht in einer Software abgebildet werden kann.

Auch US 10,105,128 B2 offenbart eine Ansteuerung einer solchen Werkzeugspitze; dort erfolgt dies über eine Mechanik, die Zahnscheiben-Segmente und Gelenkstangen umfasst, um die Bewegung der Antriebe auf die Taumelscheibe zu übertragen.

Aus der US 2005/090809 A1 ist ein Lenkgetriebe für ein chirurgisches Instrument gemäß dem Oberbegriff des Anspruchs 1 bekannt. Ferner ist aus der DE 10 2019 121092 A1 auch ein Lenkgetriebe für ein chirurgisches Instrument, das zwei motorisierte Antriebe aufweist, bekannt.

Ausgehend von diesem Stand der Technik ist es Aufgabe der vorliegenden Erfindung, ein verbessertes Lenkgetriebe für ein chirurgisches Instrument bereitzustellen, das einen Antrieb der räumlich verstellbare Taumelscheibe mit linearem Übertragungsverhalten hat und dabei platzsparend aufgebaut ist.

Diese Aufgabe wird durch ein Lenkgetriebe mit den Merkmalen des Anspruchs 1 gelöst.

Die weitere Aufgabe, ein chirurgisches Instrument bereitzustellen, dessen räumlich verstellbare Taumelscheibe durch ein konstruktiv einfaches und platzsparendes Lenkgetriebe angetrieben wird, wird durch das chirurgische Instrument mit den Merkmalen des unabhängigen Anspruchs 11 gelöst.

Weiterbildungen und bevorzugte Ausführungsformen des Lenkgetriebes und des chirurgischen Instruments sind in den Unteransprüchen ausgeführt.

Gemäß einer ersten Ausführungsform des erfindungsgemäßen Lenkgetriebes für ein chirurgisches Instrument weist dieses zwei motorisierte Antriebe auf. Es ist dazu ausgebildet, über die Stellwinkel der zwei Antriebe eine Taumelscheibe räumlich auszurichten, die dazu ausgestaltet ist, eine distale Abwinkelungsmechanik des chirurgischen Instruments zu steuern.

Erfindungsgemäß weist ein erster der zwei Antriebe einen ersten Motor auf, der über eine Antriebswelle ein Antriebsritzel antreibt, das mit einer Seitenzahnung einer ersten Zahnstange kämmt und über eine Stirnzahnung der Zahnstange ein erstes Übertragungsritzel eines ersten Doppelrades aus dem Übertragungsritzel und einem Kegelradkranz antreibt. Ferner weist ein zweiter der zwei Antriebe einen zweiten Motor auf, der über eine Antriebswelle ein Antriebsritzel antreibt, das mit einer Seitenzahnung einer zweiten Zahnstange kämmt und über eine Stirnzahnung der Zahnstange ein zweites Übertragungsritzel eines zweiten Doppelrades aus dem Übertragungsritzel und einem Kegelradkranz antreibt. Die Taumelscheibe ist dabei zwischen den beiden Kegelradkränzen, die einander zugewandt sind und auf einer gemeinsamen Achse A liegen, angeordnet.

Grundsätzlich kann eine Orientierung der Antriebsachsen bzw. der Motoren unabhängig voneinander und in Bezug auf eine durch die Längsachse B und die gemeinsamen Achse A definierte Ebene nahezu beliebig festgelegt werden, indem eine Verzahnung des Antriebsritzels mit der Seitenzahnung in Abhängigkeit der Orientierung der jeweiligen Antriebsachse entsprechend angepasst ausgebildet wird. Auf diese Weise können vorhandene Bauräume optimal ausgenutzt werden. Verlaufen die Antriebsachsen senkrecht, d. h. rechtwinklig zu der durch die Längsachse B und die gemeinsamen Achse A definierten Ebene, kann vorteilhaft - muss aber nicht - das Antriebsritzel mit der Seitenzahnung eine Geradverzahnung bilden. Weicht die Orientierung der Antriebsachsen von den 90° zu der durch die Längsachse B und die gemeinsamen Achse A definierten Ebene ab, so kann der Eingriff zwischen Antriebsritzel und Seitenzahnung beispielsweise durch eine Schrägverzahnung mit entsprechender Winkelung an Antriebsritzel und/oder Seitenzahnung bereitgestellt werden.

Durch die erfindungsgemäß ermöglichte um 90° zu der gemeinsamen Achse der Kegelradkränze versetzte Anordnung der Motoren wird in Bezug auf die Ausrichtung der Taumelscheibe horizontaler Bauraum in Richtung der gemeinsamen Achse A der Kegelradkränze eingespart.

In einer bevorzugten Ausführungsform des erfindungsgemäßen Lenkgetriebes kann eine Antriebsachse C des ersten Motors parallel zu einer Antriebsachse C' des zweiten Motors vorliegt, wobei die Antriebsachsen senkrecht, d. h. rechtwinklig, zu der gemeinsamen Achse A verlaufen. Eine sogenannte achsparallele Anordnung der Motoren ermöglicht eine kompakte und damit platzsparende Anordnung der Komponenten des Lenkgetriebes. Ferner wird durch die parallele Anordnung der Motoren eine Anordnung nah an der Hauptachse des chirurgischen Instruments erreicht und so die Kraftübertragung verbessert.

In einer weiteren Ausführungsform des erfindungsgemäßen Lenkgetriebes kann die Taumelscheibe mit einem dritten Zahnrad gekoppelt sein. Das dritte Zahnrad als Teil des Taumelscheibengetriebes steht mit den beiden Kegelradkränzen der beiden Doppelräder in Eingriff. Die Drehachse D des dritten Zahnrads steht in einem rechten Winkel zu der gemeinsamen Achse A der angetriebenen Doppelräder. Vorteilhaft wird durch die drei miteinander kämmenden Zahnräder jede Bewegung der beiden angetriebenen Zahnräder direkt auf das mit der räumlich verstellbaren Taumelscheibe gekoppelte dritte Zahnrad übertragen, sodass die Stellbewegungen der Antriebe über die auf die Taumelscheibe übertragen werden, die dadurch um die beiden gemeinsame Achse A und Drehachse D verkippt bzw. verschwenkt werden kann.

Ferner kann in einer weiteren Ausführungsform des erfindungsgemäßen Lenkgetriebes die Taumelscheibe mit einem vierten Zahnrad gekoppelt sein, das mit den beiden Kegelradkränzen der beiden Doppelräder gekoppelt und auf der abgewandten Seite von dem dritten Zahnrad angeordnet ist. Hierdurch erfolgt wird die umlaufende Verzahnungskette geschlossen und für eine gleichmäßig umlaufende und spielfreie Kraftverteilung gesorgt.

In noch einer weiteren Ausführungsform des erfindungsgemäßen Lenkgetriebes ist die Zahnstange länglich und quaderförmig, mit schmalen Stirnseiten, zwei kurzen und zwei langen; von denen eine lange Stirnseite, die Längsstirnseite, eine Stirnzahnung trägt. Entsprechend ergeben sich Seitenflächen, von denen die eine eine Seitenzahnung trägt.

Die Seitenzahnung steht mit jeweils einem Antriebsritzel in Eingriff, wobei jedes Antriebsritzel über jeweils eine Antriebswelle mit jeweils einem der Motoren verbunden ist. Die Zahnstange ist eine doppelte Zahnstange, bei der zwei Verzahnungsprofile im rechten Winkel voneinander weg weisen. Dies bewirkt eine gute und verlustarme Kraftübertragung von Motor zu den Getriebebauteilen und damit eine exakte Steuerbarkeit der Taumelscheibe.

Das Lenkgetriebe kann in einer bevorzugten Ausführungsform eine Lagerungsplatte mit je einer Führungsvorrichtung für jede Zahnstange aufweisen, die senkrecht zu der gemeinsamen Achse A und senkrecht zu den Antriebsachsen der Motoren längsbeweglich in der jeweiligen Führungsvorrichtung gelagert ist. Die Zahnstange ist mit der Stirnzahnung auf der Oberseite der Lagerungsplatte angeordnet - das ist die Seite, auf der auch die Doppelräder mit den Übertragungsritzeln angeordnet sind. Entsprechend sind die Motoren an einer Unterseite der Lagerungsplatte angeordnet. Durch die Lagerungsplatte hat die Kraftübertragungskomponente Zahnstange eine sichere Lagerung, so dass die Kraftübertragung verkantungsfrei funktionieren kann.

In einer weiteren Ausführungsform des erfindungsgemäßen Lenkgetriebes ist vorgesehen, dass die Führungsvorrichtung bzw. beide der Führungsvorrichtungen durch jeweils eine senkrecht zu der gemeinsamen Achse A und senkrecht zu den Antriebsachsen der Motoren verlaufende Führungsnut in der Lagerungsplatte bereitgestellt wird bzw. werden. Dabei ist die von der Stirnzahnung weg weisende schmale Seite der der Zahnstange mit ihrem unteren Abschnitt wie ein Steg in der Führungsnut aufgenommen. Hierdurch wird die Zahnstange in ihrer linearen Bewegung sicher geführt und die Kraftübertragung erfolgt verlustarm.

Alternativ oder besser zusätzlich kann eine Führungsvorrichtung auch durch einen an der Oberseite der Lagerungsplatte, angeordneten Führungsblock gebildet werden. Dieser ist so positioniert und gestaltet, dass er eine seitliche Führungsnut hat, die senkrecht zu der gemeinsamen Achse A und senkrecht zu den Antriebsachsen der Motoren verläuft. In dieser ist eine Längsschiene der Zahnstange aufgenommen, die an der Seitenfläche der der Zahnstange vorliegt, die von der Seitenzahnung abgewandt ist. Dies ermöglicht, dass die Zahnstange die gewünschte lineare Bewegung ausführt, ohne zu verkanten.

Noch eine weitere Ausführungsform des erfindungsgemäßen Lenkgetriebes sieht vor, dass die Seitenfläche der Zahnstangen mit der Seitenzahnung dem jeweils zugeordneten Doppelrad zugewandt ist. Die Anordnung der Verzahnungen an zwei zueinander im rechten Winkel stehenden Flächen (Seite und Schmalseite) der Zahnstange ermöglichte eine bauteilreduzierte und platzsparende Kraftübertragung der Antriebskraft der Motoren auf die Zahnräder.

Alternativ können je nach Konstruktionserfordernissen des chirurgischen Instruments oder eine Verbindung mit einem Roboter-Arm auch andere Anordnungen der Motoren bzw. der Bauteile, die die Motoren mit den Doppelrädern verbinden, vorgesehen sein. So kann die Zahnstange auch invers ausgebildet sein, so dass die Seitenzahnung auf der von den Doppelrädern abgewandten Seite der Zahnstange vorliegt und die Führungsschiene eben auf der den Doppelrädern zugewandten Seite.

Ferner kann jedes Doppelrad ein Kegelrad mit dem Kegelradkranz aufweisen, das mit dem Übertragungsritzel, das eine umlaufende Zahnung aufweist, über eine Getriebewelle verbunden ist, die sich entlang der gemeinsamen Achse A erstreckt. Aus der Kombination von Übertragungsritzel und Kegelradkranz wird eine direkte Übertragung der durch den Antrieb initiierten Bewegung auf die jeweiligen Zahnräder des Taumelscheibengetriebes erreicht.

In noch einer weiteren Ausführungsform des erfindungsgemäßen Lenkgetriebes ist die Getriebewelle in einem Lageraugenbauteil drehbar gelagert, wobei das Lageraugenbauteil auf der Lagerungsplatte befestigt ist und in Bezug auf die Drehachse A des mit der Getriebewelle in dem Lageraugenbauteil gelagerten Doppelrades ausgerichtet ist. Auch hierdurch wird im Zusammenspiel mit den weiteren Bauteilen Übertragungsritzel und Kegelradkranz eine lineare Übertragung der durch den Antrieb initiierten Bewegung auf die jeweiligen Zahnräder des Taumelscheibengetriebes erreicht.

Die Erfindung bezieht sich ferner auf ein chirurgisches Instrument, das einen Schaft, eine am proximalen Ende des Schaftes angeordnete Betätigungseinheit und ein am distalen Ende des Schaftes angeordnetes Werkzeug auf. Das Werkzeug hat eine Werkzeugspitze, die mittels einer distalen Abwinkelungsmechanik abgewinkelt werden kann. Die Abwinkelungsmechanik kann durch eine mittels zweier Antriebe räumlich ausrichtbare Taumelscheibe gesteuert bzw. ausgerichtet werden, wozu das chirurgische Instrument ein erfindungsgemäßes Lenkgetriebe aufweist, wobei die beiden Antriebe Teil des erfindungsgemäßen Lenkgetriebes sind, das dazu ausgebildet ist, die Stellwinkel der zwei Antriebe auf die räumliche Ausrichtung der Taumelscheibe zu übertragen, um so die Abwinkelungsmechanik zu steuern.

Durch das erfindungsgemäße Lenkgetriebe kann das chirurgische Instrument konstruktiv einfach und platzsparend aufgebaut werden, so dass eine einfache Verbindung zu einem Roboter-Arm ermöglicht werden kann, bei der die Bewegung der Antriebe linear auf die Werkzeugspitze übertragen werden kann. Folge ist eine exakt steuerbare Verwendung des chirurgischen Instruments.

Um die räumlich verstellbare Taumelscheibe trotz der drehfesten Kopplung mit dem dritten Zahnrad, das mit den beiden Kegelradkränzen der beiden Doppelräder in Eingriff steht, dreidimensional verstellen zu können, d. h. die Kipp- bzw. Schwenkbewegungen mit einer Rotation der Taumelscheibe um die Längsachse überlagern zu können, kann eine bevorzugte Ausführungsform des chirurgischen Instruments vorsehen, dass die Taumelscheibe um die Längsachse B des Schaftes über einen Lagerring rotierbar in einem Lenkring gelagert ist, der drehfest mit dem dritten Zahnrad gekoppelt ist. Zur rotativen Kopplung der Taumelscheibe mit einer koaxial zu einer Längsachse B des Schaftes verlaufenden Hauptwelle kann die Taumelscheibe kardanisch mit der Hauptwelle gekoppelt sein. Somit kann die Werkzeugspitze mittels der Taumelscheibe zusätzlich zum Verschwenken bzw. Verkippen durch die beiden Antriebe durch die Hauptwelle relativ zur Längsachse des Schaftes um die Längsachse des Schaftes rotiert werden, ohne dass die Lenkdrähte verdrillt werden.

Zur Ausbildung der kardanischen Lagerung der räumlich verstellbaren Taumelscheibe kann eine Ausführungsform des erfindungsgemäßen chirurgischen Instruments vorsehend, dass die Taumelscheibe über zwei um 180 ° versetzt zueinander angeordnete Lagerstifte verschwenkbar auf einer Kreuzgelenkscheibe gelagert ist, wobei die Kreuzgelenkscheibe über zwei um 180 ° versetzt zueinander angeordnete Lagerstifte verschwenkbar auf der Hauptwelle gelagert ist und wobei die Lagerstifte der Taumelscheibe und der Kreuzgelenkscheibe um 90 ° versetzt zueinander angeordnet sind. Die kardanische Aufhängung ermöglicht eine Bewegungsführung in allen drei Raumachsen, wodurch die Werkzeugspitze gezielt gesteuert werden kann. Alternativ zu einer Kreuzgelenkscheibe mit zwei rechtwinklig gekreuzten Stift-Paaren zur kardanischen Lagerung der Taumelscheibe auf der Hauptwelle kann eine vorteilhafte Ausführungsform vorsehen, dass zur kardanischen Lagerung die Hauptwelle zwei in ihrer Außenfläche vorliegende Führungsnuten aufweist, die sich diametral und längs der Hauptwelle erstrecken, wobei die Taumelscheibe, die kreisringförmig mit einer Außenseite und einer Innenseite ausgebildet ist, zwei diametral und radial nach innen weisend an der Taumelscheibe angeordnete Stifte aufweist. Jeder der zwei fest an bzw. in der Taumelscheibe montierten Stifte greift in eine der beidseitig in die Hauptwelle eingebrachten Führungsnuten ein, sodass ein Drehwinkel der Welle auf die Taumelscheibe übertragbar ist. Vorteilhaft ergibt sich so eine drehsteife Verbindung zwischen Hauptwelle und Taumelscheibe, die auch bei einem großen Winkelversatz (± 40° und mehr) und Axialversatz eine Drehwinkelübertragung erlaubt, und dabei sehr kompakt aufgebaut, sowie einfach herzustellen und zu montieren ist. Grundsätzlich können allerdings auch eine Bogenzahnkupplung trotz eines relativ geringen Winkelversatzes, ein Gleichlaufgelenk trotz der aufwändigen Fertigung und komplexen Montage oder eine stoffschlüssige Kupplung, die häufig mit einer spielbehafteten Drehwinkelübertragung verbunden ist, zur kardanischen Lagerung einer Taumelscheibe auf einer Hauptwelle eingesetzt werden.

Ferner sieht eine weitere Ausführungsform des erfindungsgemäßen chirurgischen Instruments vor, dass das vierte Zahnrad mit der Taumelscheibe über einen Lagerring mit dem Lenkring gekoppelt ist, wobei das vierte Zahnrad gegenüber dem dritten Zahnrad frei drehbar ist. Dieses vierte Zahnrad schließt die umlaufende Verzahnungskette und sorgt so für eine gleichmäßig umlaufende und spielfreie Kraftverteilung.

In einer weiteren Ausführungsform des erfindungsgemäßen chirurgischen Instruments ist ein Betätigungselement axial verschiebbar in dem Schaft gelagert und steht proximalseitig mit der Betätigungseinheit in Wirkverbindung. Die distale Abwinkelungsmechanik der abwinkelbaren Werkzeugspitze besteht aus an dem distalen Ende des Schaftes angeordneten Schwenkgliedern, die über die in Längsrichtung des Schaftes verlaufenden Lenkdrähte mit der Taumelscheibe des Lenkgetriebes verbunden sind. Die Lenkdrähte können an der Taumelscheibe mittels einer Klemmverbindung klemmend gelagert, damit in einem Fall der Beschädigung die Lenkdrähte einfach ausgetauscht werden können. Alternativ können die Lenkdrähte beispielsweise auch durch Schweißen oder Crimpen an der Taumelscheibe befestigt sein.

In einer weiteren Ausführungsform des erfindungsgemäßen chirurgischen Instruments ist ein radialer Abstand der Lenkdrähte von der Längsachse des Schaftes an der Taumelscheibe größer als am proximalen Ende des Schaftes, aus dem die Lenkdrähte austreten. Dabei können die Lenkdrähte sich von dem proximalen Ende des Schaftes direkt zu der Taumelscheibe erstrecken, wobei die Lenkdrähte unter einem von 90° abweichenden Winkel zur Taumelscheibe verlaufen. Alternativ kann distalseitig vor der Taumelscheibe eine Fächerscheibe auf der Hauptwelle angeordnet sein, die den radialen Abstand der aus dem proximalen Schaftende austretenden Lenkdrähte von der Längsachse des Schaftes vergrößert, sodass die Lenkdrähte zwischen der Fächerscheibe und der Taumelscheibe annährend parallel zueinander verlaufen und in Bezug auf eine Scheibenfläche der Taumelscheibe einen Winkel von ca. 90° bilden. Aufgrund des geringeren Bauraumbedarfs kann die Variante ohne Fächerscheibe bevorzugt sein. Durch die Vergrößerung des radialen Abstandes der Lenkdrähte von der Längsachse des Schaftes, von beispielsweise einem Durchmesser von 4 mm auf einen Durchmesser von 18 mm, werden nicht nur die Montage und Fertigung des mit der räumlich verstellbaren Scheibe ausgestatten Antriebs der Lenkdrähte vereinfacht, sondern auch die Verstellwinkel der räumlich verstellbaren Scheibe bzw. infolge des vergrößerten Hebels die zur Abwinkelung benötigten Kräfte verringert, um einen dem Maß der Durchmesservergrößerung entsprechenden Verschwenkwinkel der Werkzeugspitze zu erzielen.

Um beim Verschwenken des dritten und vierten Zahnrads relativ zur Längsachse des Schaftes eine Kollision der Zahnräder mit den Lenkdrähten und gegebenenfalls dem Betätigungselement zu vermeiden, können in den Zahnkränzen des dritten Zahnrads und des vierten Zahnrads Aussparungen für die Lenkdrähte und das Betätigungselement ausgebildet sein.

Das erfindungsgemäße chirurgische Instrument hat den Vorteil, dass viele dünne Lenkdrähte zur Ansteuerung der verschwenkbaren Werkzeugspitze verwendet werden können und diese Ansteuerung aufgrund des motorisierten Antriebs für die räumlich verstellbare Scheibe, an der die Lenkdrähte proximalseitig gelagert sind, feinfühlig, exakt und reproduzierbar erfolgt.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Dabei zeigen:
- Fig. 1: eine perspektivische Ansicht des chirurgischen Instruments mit schematisch dargestellter Betätigungseinheit,
- Fig. 2: eine perspektivische Detailansicht einer ersten Ausführungsform des erfindungsgemäßen Lenkgetriebes,
- Fig. 3: eine Detailansicht der Zahnstange,
- Fig. 4: eine perspektivische Ansicht eines erfindungsgemäßen Lenkgetriebes gemäß einer weiteren Ausführungsform,
- Fig. 5: eine perspektivische, teilweise ausgeschnittene Detailansicht des Lenkgetriebes aus Fig. 4, und
- Fig. 6: eine perspektivische, teilweise ausgeschnittene Detailansicht des Taumelscheibengetriebes gemäß einer weiteren Ausführungsform.

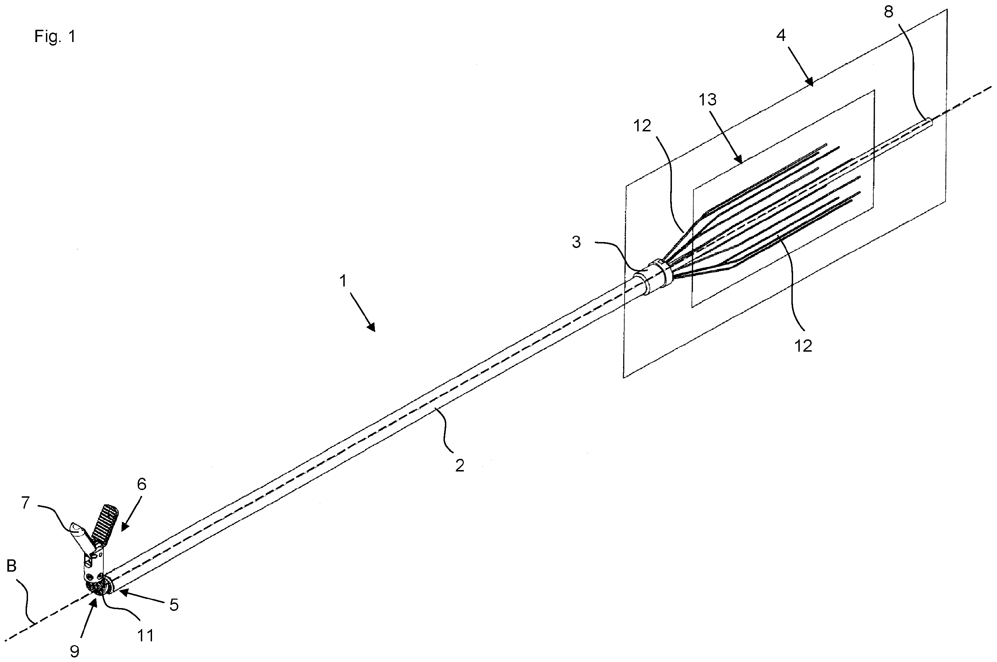

In Fig. 1 ist ein chirurgisches Instrument 1 mit einem hohlen Schaft 2 gezeigt, das eine am proximalen Ende 3 des Schaftes 2 angeordnete, schematisch dargestellte Betätigungseinheit 4 und eine am distalen Ende 5 des Schaftes 2 angeordnete Werkzeugspitze 6 aufweist. Die Werkzeugspitze 6 ist mit einem Instrument 7 verbunden, wobei das Instrument 7 über ein axial verschiebar im Schaft 2 gelagertes Betätigungselement 8 betätigbar ist, das proximalseitig mit der Betätigungseinheit 4 in Wirkverbindung steht. Bei der Betätigungseinheit 4 kann es sich um eine manuell betätigbare Handhabe oder aber um eine für den robotischen Einsatz ausgelegte, also auch ohne manuelles Zutun betätigbare Baueinheit handeln.

Bei dem Instrument 7 der Werkzeugspitze 6 kann es sich beispielsweise um ein mit Maulteilen versehenes Werkzeug, wie in Fig. 1 dargestellt, oder aber um ein Endoskop, einen Applikator oder dergleichen handeln.

Die Werkzeugspitze 6 ist über einen Gelenkmechanismus 9 relativ zur Längsachse B des Schaftes 2 verschwenkbar, wobei der Gelenkmechanismus 9 aus am distalen Ende des Schaftes 5 angeordneten Schwenkgliedern 11 besteht, die über in Längsrichtung des Schaftes 2 verlaufende Lenkdrähte 12 so mit einem am proximalen Ende 3 des Schaftes 2 angeordneten Antrieb 13 verbunden sind, dass eine Bewegung des proximalseitigen Antriebs 13 eine entsprechende relative Bewegung der distalseitigen Schwenkglieder 11 und somit ein Verschwenken der Werkzeugspitze 6 verursacht.

Auch wenn voranstehend und nachfolgend nur der Begriff Lenkdrähte 12 verwendet wird, können funktional auch Lenkseile verwendet werden, weshalb der verwendete Begriff Lenkdrähte 12 synonym auch als Lenkseil zu lesen und zu verstehen ist.

Das axialverschiebbar im Schaft 2 gelagerte Betätigungselement 8 zum Betätigen des beispielsweise aus zwei Maulteilen bestehenden Instruments 7 ist bei den dargestellten Ausführungsformen als Zug-/Schubstange ausgebildet.

Der Antrieb 13 für die Lenkdrähte 12 ist bei dem in den Abbildungen dargestellten und nachfolgend beschriebenen chirurgischen Instrument 1 als motorisierter Antrieb 13 ausgebildet.

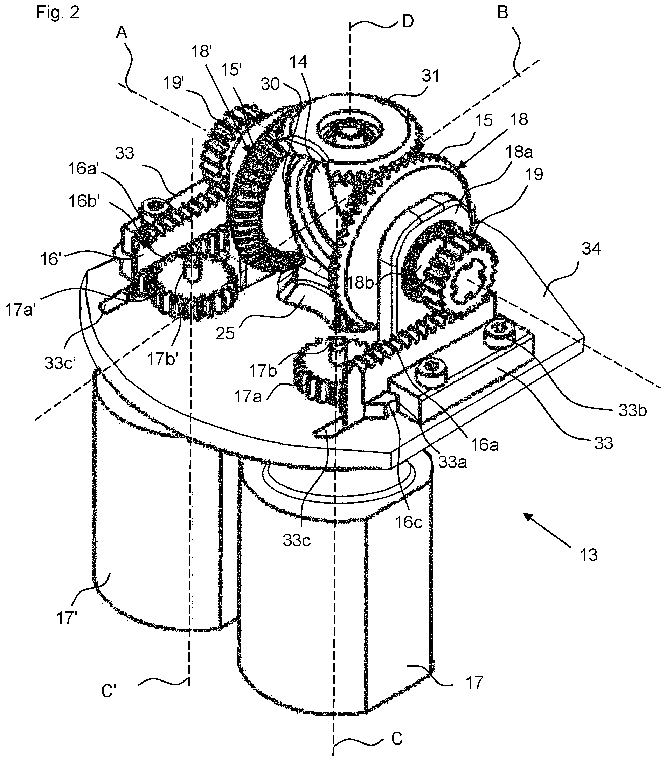

Kernstück des Antriebs 13 ist eine räumlich verstellbare Taumelscheibe 14 (Fig. 2, 4 bis 6), an der die Lenkdrähte 12 so gelagert sind, dass eine Verlagerung der Taumelscheibe 14 über die an ihr gelagerten Lenkdrähte 12 ein Verschwenken der Werkzeugspitze 6 bewirkt. Mittels des motorisierten Antriebs 13 kann die Taumelscheibe 14 verlagert werden.

Durch die Verwendung eines motorisierten Antriebs 13 für die Taumelscheibe 14 ist es möglich, die Lenkdrähte 12 zum Verschwenken der distalseitigen Schwenkglieder 11 bzw. der Werkzeugspitze 6 exakt, feinfühlig in kleinsten Schritten und auch reproduzierbar anzusteuern. Darüber hinaus ist die Anzahl der zu verwendenden Lenkdrähte 12 für ein motorisiertes Lenkgetriebe 13 recht frei wählbar.

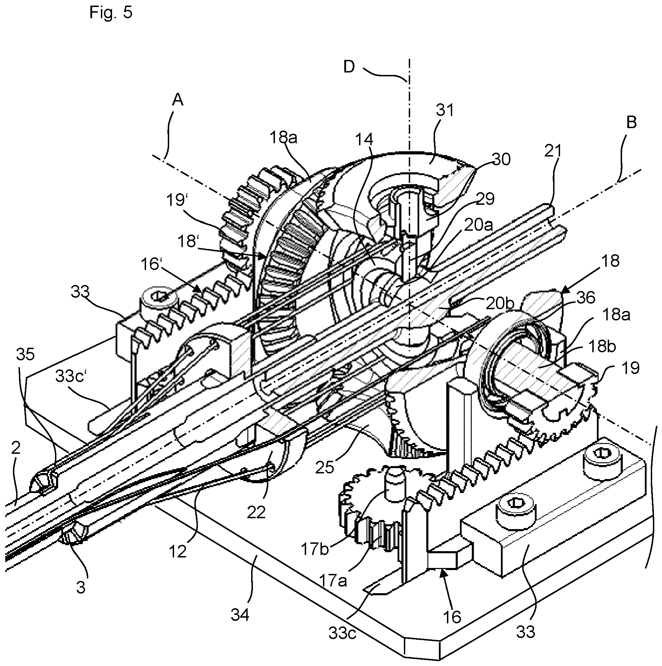

In Fig. 2 ist das Lenkgetriebe 13 vereinfacht dargestellt, wobei das Lenkgetriebe 13 in der Mitte die Taumelscheibe 14 aufweist. Mit der Taumelscheibe 14 sind vier Zahnräder verbunden, die diametral zu der Taumelscheibe 14 angeordnet sind. Unterhalb und oberhalb der Taumelscheibe 14 in Fig. 2 sind ein drittes Zahnrad 25 und ein viertes Zahnrad 31 angeordnet und operativ mit der Taumelscheibe 14 gekoppelt. D. h., eine Bewegung eines dieser Zahnräder 25, 31 hat eine direkte Bewegung der Taumelscheibe 14 zur Folge. In die Zahnräder 25, 31 greifen jeweils zu einer linken und rechten Seite angeordneten Doppelräder 18, 18' ein. Hierzu weisen die Doppelräder 18, 18' Kegelradkränze 15, 15' auf, die direkt in die Zahnkränze des dritten Zahnrads 25 und des vierten Zahnrads 31 eingreifen. Die Doppelräder 18, 18' weisen in entgegengesetzte Richtungen entlang ihrer Mittelachse A, die auch die gemeinsame Drehachse beider Doppelräder 18, 18' bildet, Übertragungsritzel 19, 19' auf. Diese Übertragungsritzel 19, 19' sind mit einer Getriebewelle 18b verbunden, die über einen Lagerring 36 (vgl. Fig. 5) drehbar in einem Lageraugenbauteil 18a gelagert ist. Die Getriebewelle 18b verbindet die beiden Bauteile Kegelradkranz 15, 15' und Übertragungsritzel 19, 19', wodurch je ein Doppelrad 18, 18' gebildet wird. Dies gilt für beide Doppelräder 18,18'. Die Übertragungsritzel 19, 19' wirken jeweils mit einer Zahnstange 16, 16' zusammen, wobei die Übertragungsritzel 19, 19' jeweils in eine Stirnverzahnung 16a, 16a' der Zahnstange 16 greifen.

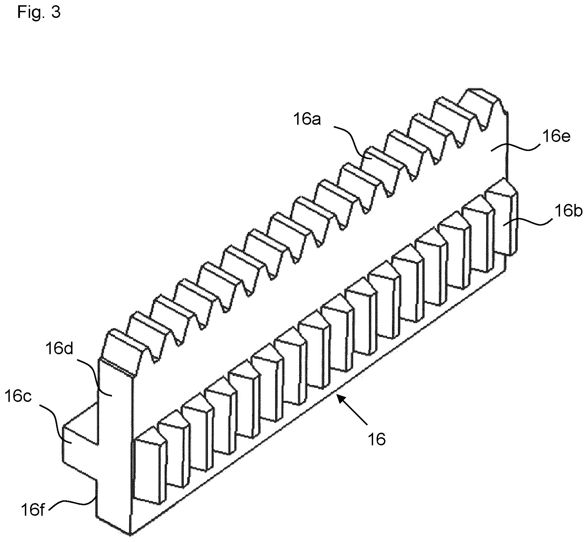

Die Zahnstange 16 ist in Fig. 3 einzeln dargestellt. Die Zahnstange 16 ist länglich und quaderförmig mit schmalen, kurzen Stirnseiten 16d und langen Stirnseiten bzw. Längsstirnseiten, von denen die eine die Stirnverzahnung 16a trägt und die von ihr abgewandte Seite 16f als "Steg" wirkt, der in einer Führungsnut 33c, siehe unten, der Führungsvorrichtung aufgenommen werden kann.

Entlang der Seitenfläche 16e (in Fig. 3 nach vorne weisend) ist entlang der Länge der Zahnstange eine seitliche Zahnung 16b angeordnet. An der von der Seitenzahnung 16b abgewandten zweiten Seitenfläche ist eine Schiene (auch als Steg anzusehen) 16c ausgebildet, die sich ebenfalls entlang der gesamten Länge der Zahnstange 16 erstreckt.

Die Zahnstange 16 (und das gilt auch für die zweite Zahnstange 16') ist in dem Lenkgetriebe 13 in einer Führungsnut 33c beweglich gelagert, die in eine Lagerungsplatte 34 eingelassen ist. In dieser Führungsnut 33c ist die Zahnstange 16 mit ihrer unteren Schmalseite 16f ein Stück weit aufgenommen und somit gelagert.

Die seitliche Schiene 16c liegt auf oder oberhalb der Verbindungplatte 34 und verläuft in der Führungsnut 33a eines Führungsblocks 33, der über Befestigungsmittel 33b, wie bspw. Verschraubungen, an der Oberseite der Lagerungsplatte 34 befestigt ist.

Angetrieben werden die Zahnstangen 16, 16' durch Motoren 17, 17' über an Antriebswellen 17b' 17b' der Motoren 17, 17' befestigten Antriebsritzeln 17a, 17a', deren Drehachse einer Drehachse C, C' der Motoren 17, 17' entspricht. Durch Drehen des Antriebsritzels 17a, 17a' wird die Zahnstange 16, 16' in den Nuten 33c, 33a linear entlang der Drehrichtung, d. h. in Tangentialrichtung des Übertragungsritzels 19, 19' senkrecht zur Drehachse A und senkrecht zu den Antriebsachsen C, C' bewegt. Die tangentiale Drehrichtung des Übertragungsritzels 19, 19' am Eingriff mit der Zahnstange 16,16' in Fig. 2, entsprechend der Bewegungsrichtung der Zahnstange 16, ist parallel zu der Instrumentenachse B.

So wird die Rotation der Motoren 17,17' und damit der Antriebsritzel 17a, 17a', entsprechend den Drehachsen C, C' der Motoren 17, 17', von der Seitenzahnung 16b, 16b' der Zahnstange 16, 16' übernommen und auf eine lineare Bewegung der Zahnstange 16, 16' parallel zu der Instrumentenachse B übertragen. In Fig. 2 sind diese Drehachsen C, C' parallel zu einer Drehachse D des dritten Zahnrads 25 und des vierten Zahnrads 31. Die lineare Bewegung der Zahnstange 16, 16' wird von dem Übertragungsritzel 19, 19' aufgenommen und in eine Drehbewegung des jeweiligen Doppelrades 18, 18' um deren Drehachse A umgesetzt. Die Drehbewegung der Doppelräder 18, 18' bewirkt dann eine Drehbewegung des dritten Zahnrads 25 bzw. des vierten Zahnrads 31 um deren Drehachse D, die im rechten Winkel zu der gemeinsamen Achse A der Doppelräder 18, 18' liegt, und damit eine Bewegung der Taumelscheibe 14.

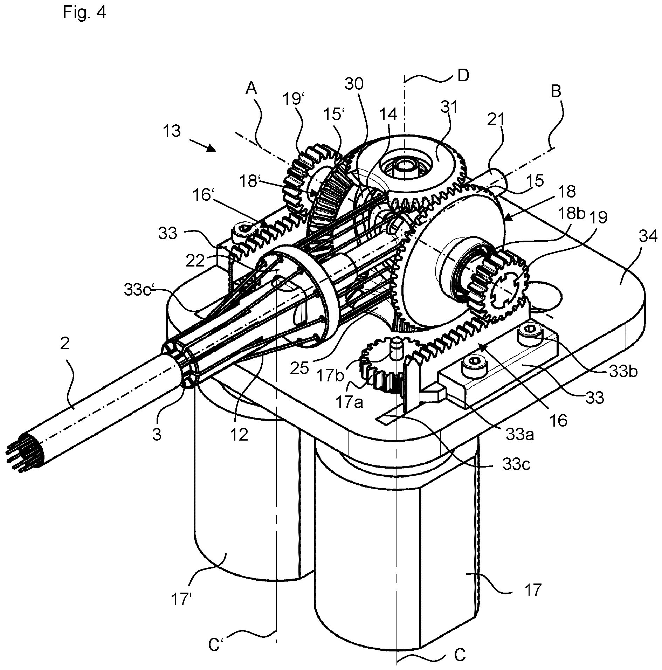

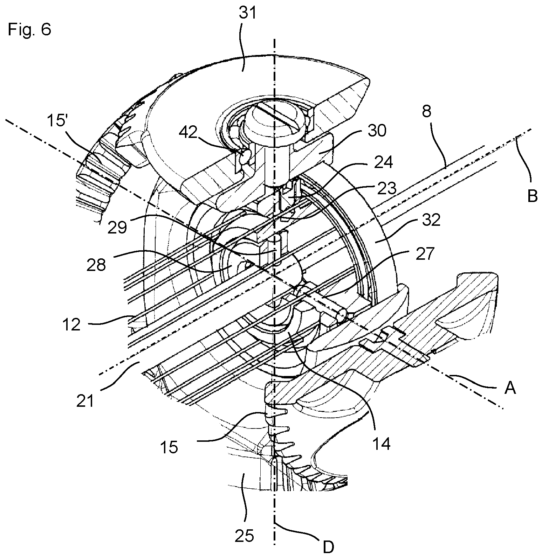

Der Aufbau und der Betrieb des Lenkgetriebes 13 in Bezug auf die Ansteuerung der über die Antriebseinheiten betätigbaren Taumelscheibe 14 und deren Lagerung werden nachfolgend anhand Fig. 4 bis 6 beschrieben, wobei in Fig. 6 aus Gründen der besseren Übersichtlichkeit nur die Kegelräder mit den Kegelradkränzen 15, 15' der Doppelräder 18, 18' dargestellt sind.

Im Schaft 2 des Instruments 1 ist eine sich koaxial zur Längsachse B des Schaftes 2 erstreckende hohle Hauptwelle 21 angeordnet, die um die Längsachse B des Schaftes 2 rotierbar ist und sich über das proximale Ende 3 des Schaftes 2 hinaus bis in den Bereich des Lenkgetriebes 13 erstreckt. Innerhalb dieser hohlen Hauptwelle 21 ist axialverschiebbar das Betätigungselement 8 zur Betätigung des Instruments 7 gelagert.

Die Lenkdrähte 12, die am proximalen Ende 3 des Schaftes 2 aus dem Schaft 2 austreten, wofür am proximalen Schaftende ein Schaftendstück 3 vorgesehen sein kann, in dem Durchtrittschlitze 33 für die Lenkdrähte 12 vorgesehen sind, werden in den dargestellten Beispielen über eine drehfest in Bezug auf der Hauptwelle 21 an dem Schaftendstück 3 angeordnete Fächerscheibe 22 aufgefächert, wodurch der radiale Abstand der Lenkdrähte 12 von der Längsachse B des Schaftes 2 vergrößert wird. Während der Durchmesser des die Längsachse B des Schaftes 2 koaxial umgebenden Bündels der Lenkdrähte 12 innerhalb des Schaftes 2 bzw. an dem distalen Ende 5 im Bereich der Abwinkelungsmechanik 9 beispielsweise 4 mm beträgt, beträgt der Durchmesser des von den Lenkdrähten 12 gebildeten Bündels hinter der Fächerscheibe 22 beispielsweise 18 mm. Durch die mit Hilfe der Fächerscheibe 22 erzielte Vergrößerung des radialen Abstandes der Lenkdrähte 12 von der Längsachse B des Schaftes 2 werden nicht nur die Montage und Fertigung des mit der Taumelscheibe 14 ausgestatten Getriebes 13 vereinfacht, sondern wird auch der notwendige Verstellwinkel der Taumelscheibe 14 proportional verringert, um einen erwünscht hohen Verschwenkwinkel der Werkzeugspitze 6 zu erzielen. Mit der beispielhaft beschriebenen Vergrößerung des Durchmessers des Lenkdrahtbündels von 4 mm innerhalb des Schaftes 2 auf 18 mm hinter der Fächerscheibe 22 verringert sich der Verstellwinkel der Taumelscheibe 14 entsprechend um das 4,5-fache gegenüber dem am distalen Ende erzielbaren Verschwenkwinkel der Werkzeugspitze 6. Um diese um 90°abzuwinkeln, bedarf es somit nur einer Verschwenkung der Taumelscheibe 14 um 20°.

Proximalseitig hinter der Fächerscheibe 22 werden die parallel zur Längsachse B des Schaftes 2 verlaufenden Lenkdrähte 12 der Taumelscheibe 14 zugeführt. In einer nicht dargestellten Alternative verlaufen können die am proximalen Ende 3 austretenden Lenkdrähte 12 direkt ohne Fächerscheibe zur Taumelscheibe 14 verlaufen, sodass die Lenkdrähte unter einem Winkel zur Längsachse B der Taumelscheibe 14 zugeführt werden. Zum Festlegen der Lenkdrähte 12 an der Taumelscheibe 14 sind in der Taumelscheibe 14 Durchgangsbohrungen 23 für jeden Lenkdraht 12 ausgebildet, wobei im dargestellten Beispiel die Lenkdrähte 12 innerhalb der Durchgangsbohrungen 23 über Madenschrauben 24 kraftschlüssig mit der Taumelscheibe 14 verbunden und fixiert sind. Dazu alternative Befestigungsformen der Lenkdrähte an der Taumelscheibe umfassen beispielsweise auch Schweißen oder Crimpen oder andere Klemmvorrichtungen.

Die Doppelräder 18 und 18' als Antriebsräder sind mit dem dritten Zahnrad 25 gekoppelt, das vorzugsweise als Kegelrad ausgebildet ist und mit den beiden Kegelradkränzen 15, 15' der Doppelräder 18 und 18' in Eingriff steht, sodass die Drehachse D des dritten Zahnrads 25 die gemeinsame Drehachse A der Doppelräder 18 und 18' sowie die Längsachse B des Schaftes 2 schneidet. Durch die drei miteinander kämmenden Zahnräder 18, 18' und 25 wird jede Bewegung der beiden Doppelräder 18 und 18' direkt auf die mit dem dritten Zahnrad 25 gekoppelte Taumelscheibe 14 übertragen, was eine direkte Betätigung der Lenkdrähte 12 bewirkt.

Zur Ausbildung einer kardanischen Lagerung der Taumelscheibe 14 auf der Hauptwelle 21 ist die Taumelscheibe 14 über zwei um 180° versetzt zueinander angeordnete Lagerstifte 27 verschwenkbar auf einer Kreuzgelenkscheibe 28 gelagert, die wiederrum über zwei um 180° versetzt zueinander angeordnete Lagerstifte 29 verschwenkbar auf der Hauptwelle 21 gelagert ist. In Fig. 6 ist aufgrund der Teilschnittansicht jeweils nur ein Lagerstift 27 und ein Lagerstift 29 zu sehen.

Die Lagerstifte 27 der Taumelscheibe 14 und die Lagerstifte 29 der Kreuzgelenkscheibe 28 sind dabei um 90° versetzt zueinander angeordnet. Diese Lagerung ermöglicht es, die Taumelscheibe 14 um zwei rechtwinklig zueinander stehende Achsen relativ zur Längsachse B des Schaftes 2 zu verschwenken und eine Rotation der Hauptwelle 21 um die Längsachse B auf die Taumelscheibe 14 zu übertragen, wodurch über die Lenkdrähte 12 distalseitig die Werkzeugspitze 6 (vgl. Fig. 1) in alle Raumrichtungen relativ zur Längsachse B des Schaftes 2 verschwenkbar ist.

Eine alternative kardanische Lagerung der Taumelscheibe 14 auf der Hauptwelle 21 weist das in Fig. 4 und 5 dargestellte Lenkgetriebe 13 auf. Diese konstruktiv einfachere, kompakter aufgebaut und einfacher zu montiere Lagerungsanordnung ermöglicht es ebenfalls, die Taumelscheibe 14 um zwei Freiheitsgrade zu verschwenken und um die Längsachse B zu rotieren, wodurch über die Lenkdrähte 12 distalseitig die Werkzeugspitze 6 in alle Raumrichtungen relativ zur Längsachse B des Schaftes 2 verschwenkbar ist. Dabei weist die Hauptwelle 21 in dem zur Lagerung der Taumelscheibe 14 vorgesehenen Bereich zwei sich längs der Hauptwelle 21 erstreckende und beidseitig bzw. diametral in der Hauptwelle 21 eingebrachte Führungsnuten 20a auf, in die zwei diametral und radial nach innen weisend an der Taumelscheibe 14 angeordnete Stifte 29 eingreifen, wobei in Fig. 5 nur eine Führungsnut 20a mit dem der darin eingreifenden Stift 29 zu sehen ist. Durch diesen Eingriff kann die Taumelscheibe 14 aus einer neutralen Position, in der die Taumelscheibe 14 in einer durch die Drehachse A definierten Ebene senkrecht, d. h. rechtwinklig, zur Längsachse B liegt, sowohl um Drehachse D als auch um Drehachse A verschwenkt werden. Überlagerte Bewegungen durch Verschwenken um beide Drehachsen A, D, sind ebenfalls möglich. Ferner gestattet der Eingriff der Stifte 29 in die Führungsnuten 20a die Übertragung eines Drehwinkels der Hauptwelle 21 auf die Taumelscheibe 14, sodass die Taumelscheibe 14 dreidimensional relativ zur Längsachse B des Schaftes 2 verlagert werden kann. Die maximale Verkippung bzw. Verdrehung bzw. die maximalen Kipp- und Drehwinkel um Drehachse A und D sind durch die Länge und Tiefe der Führungsnuten 20a im Zusammenwirken mit Innendurchmesser und Stärke der Taumelscheibe 14 und Länge der Stifte 29 bestimmt. Im dargestellten Beispiel weist die Hauptwelle 21 in dem zur Lagerung der Taumelscheibe 14 vorgesehenen Bereich einen Kugelabschnitt 20b auf, an dem die Führungsnuten 20a vorliegen und der die Taumelscheibe 14 in axialer Richtung festlegt. Die Taumelscheibe 14 weist hierbei eine an den Kugelabschnitt 20b angepasst konturierte Aufnahmeausnehmung auf.

Wie weiterhin aus Fig. 2 und 4 bis 6 ersichtlich, ist die räumlich verstellbare Taumelscheibe 14 in einem drehfest mit dem dritten Zahnrad 25 gekoppelten Lenkring 30 gelagert. Um die durch die Doppelräder 18, 18' und Zahnrad 25 gebildete Verzahnungskette zu einem geschlossenen Verzahnungsring zu schließen, der eine gleichmäßig umlaufende Kraftverteilung gewährleistet, ist auf der Drehachse D des dritten Zahnrads 25 gegenüber liegend zum dritten Zahnrad 25 das vierte Zahnrad 31 angeordnet, das ebenfalls vorzugsweise als Kegelrad ausgebildet ist mit den Kegelradkränzen 15, 15' der beiden Doppelräder 18 und 18' in Eingriff steht.

Die Taumelscheibe 14 ist über einen Lagerring 32 (vgl. Fig. 6) in dem drehfest mit dem dritten Zahnrad 25 gekoppelten Lenkring 30 gelagert, um eine Rotation der Taumelscheibe 14 um die Längsachse B des Schaftes 2 zu ermöglichen. Der drehfest mit dem dritten Zahnrad 25 gekoppelte Lenkring 30 ist durch eine Lagerung mittels Lagerring 42 frei in Bezug auf das vierte Zahnrad 31 drehbar, so dass eine Rotation des vierten Zahnrads 31 um seine Drehachse D keine Verdrehung des Lenkrings 30 und der Taumelscheibe 14 bewirkt.

Die beschriebene kardanische Lagerung der Taumelscheibe 14 auf der Hauptwelle 21 ermöglicht es, die Taumelscheibe 14 dreidimensional relativ zur Längsachse B des Schaftes 2 zu verlagern. Wenn ausgehend von der in Fig. 4 bis 6 dargestellten neutralen Ausgangsstellung, in der die Taumelscheibe 14 senkrecht zur Längsachse B des Schaftes 2 ausgerichtet ist, die Doppelräder 18 und 18' über die Motoren 17, 17'so angetrieben werden, dass sich die Doppelräder 18 und 18' in dieselbe Richtung drehen, bewirkt diese Verdrehung der Doppelräder 18 und 18' aufgrund des kämmenden Eingriffs mit dem dritten Zahnrad 25 und dem vierten Zahnrad 31 ein Verkippen der Baueinheit, die aus dem dritten Zahnrad 25, der mit dem dritten Zahnrad 25 über den Lenkring 30 gekoppelten Taumelscheibe 14 und dem vierten Zahnrad 31 gebildet wird, um die gemeinsame Drehachse A der Doppelräder 18 und 18'.Zur Vereinfachung der Funktionsbeschreibung wird nachfolgend auf die Ausrichtung der Lagerstifte 27, 29 der kardanischen Lagerung in Bezug auf die Drehachsen A und D genommen. Tatsächlich werden bei Rotation der Hauptwelle 21 und damit der Taumelscheibe 14 die Lagerstifte 27, 29 nicht mehr wie dargestellt mit den Achsen A und D fluchten, sodass die durch die Lagerstifte 27, 29 bereitgestellten Schwenkachsen der Taumelscheibe 14 von den Drehachsen A, D des Lenkgetriebes 13 abweichen können.

Im Beispiel von Fig. 6 ermöglichen die mit der Drehachse A der Doppelräder 18 und 18' fluchtenden Lagerstifte 27, über die die Taumelscheibe 14 verschwenkbar an der Kreuzgelenkscheibe 28 gelagert ist, dieses Verkippen der Taumelscheibe 14 relativ zur Hauptwelle 21. Dieses Verkippen der Taumelscheibe 14 um die Drehachse A relativ zur Längsachse B des Schaftes 2 bewirkt über die Lenkdrähte 12, dass distalseitig die Werkzeugspitze 6 in entsprechender Weise relativ zur Längsachse B des Schaftes 2 verschwenkt wird. Im Beispiel von Fig. 4 und 5 wird das Verkippen der Taumelscheibe 14 um die Drehachse A durch die Bewegung der Führungsstifte 29 in den Führungsnuten 20a im Kugelabschnitt 20b der Hauptwelle 21 ermöglicht.

Wenn ausgehend von der in Fig. 4 bis 6 dargestellten neutralen Ausgangsstellung, in der die Taumelscheibe 14 senkrecht zur Längsachse B des Schaftes 2 ausgerichtet ist, die Doppelräder 18 und 18' über die Motoren 17, 17' so angetrieben werden, dass sich die Doppelräder 18 und 18' in entgegengesetzte Richtungen drehen, bewirkt diese Verdrehung der Doppelräder 18 und 18' aufgrund des kämmenden Eingriffs mit dem dritten Zahnrad 25 ein Verdrehen der Baueinheit, die aus dem dritten Zahnrad 25, und der mit dem dritten Zahnrad 25 über den Lenkring 30 gekoppelten Taumelscheibe 14 gebildet wird, um die Drehachse D des dritten Zahnrads 25.

Im Beispiel von Fig. 6 ermöglichen die mit der Drehachse D des dritten Zahnrads 25 fluchtenden Lagerstifte 29, über die die Kreuzgelenkscheibe 28 verschwenkbar an der Hauptwelle 21 gelagert ist, zusammen mit der freien Drehbarkeit der Taumelscheibe 14 relativ zum vierten Zahnrad 31 aufgrund des Lenkrings 32 dieses Verdrehen der Kreuzgelenkscheibe 28 relativ zur Hauptwelle 21. Dieses Verdrehen der Taumelscheibe 14 um die Drehachse D relativ zur Längsachse B des Schaftes 2 bewirkt über die Lenkdrähte 12 das distalseitig die Werkzeugspitze 6 in entsprechender Weise relativ zur Längsachse B des Schaftes 2 verschwenkt wird. Analog wird im Beispiel von Fig. 4 und 5 das Verdrehen der Taumelscheibe 14 um die Drehachse D durch die in die Führungsnuten 20a eingreifenden und mit der Drehachse D des dritten Zahnrads 25 fluchtenden Stifte 29 bewirkt.

Es ist selbstverständlich möglich, die beschriebenen Bewegungen zu überlagern, sodass beispielsweise die Taumelscheibe 14 um die gemeinsame Drehachse A der Doppelräder 18, 18' verkippt und gleichzeitig auch noch um die Drehachse D des dritten Zahnrads 25 verdreht wird. Durch die Kombination der beiden Bewegungsabläufe aufgrund der einzeln ansteuerbaren Motoren 17, 17' des Getriebes 13 und die Kopplung mit der Hauptwelle 21 lässt sich die Taumelscheibe 14 dreidimensional relativ zur Längsachse B des Schaftes 2 verstellen, woraus aufgrund der Kopplung über die Lenkdrähte 12 eine entsprechende räumliche Verlagerung der Werkzeugspitze 6 resultiert.

Ein wie zuvor beschrieben ausgebildetes chirurgisches Instrument 1 zeichnet sich dadurch aus, dass viele dünne Lenkdrähte 12 zur Ansteuerung der verschwenkbaren Werkzeugspitze 6 verwendet werden können, und diese Ansteuerung auf Grund des motorisierten Antriebs 13 für die Taumelscheibe 14, an der die Lenkdrähte 12 gelagert sind, feinfühlig, exakt und reproduzierbar erfolgt.

In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen, im Umfang der beigefügten Ansprüche. Die vorliegende Erfindung stellt ein Lenkgetriebe 13 für ein chirurgisches Instrument 1 bereit, wobei das Lenkgetriebe 13 zwei motorisierte Antriebe aufweist und dazu ausgebildet ist, über die Stellwinkel der zwei Antriebe eine Taumelscheibe 14 räumlich auszurichten, die dazu ausgebildet ist, eine distale Abwinkelungsmechanik 9 des chirurgischen Instruments 1 zu steuern. Dabei weist ein erster der zwei Antriebe einen ersten Motor 17 auf, der über eine Antriebswelle 17b ein Antriebsritzel 17a antreibt, das mit einer Seitenzahnung 16b einer ersten Zahnstange 16 kämmt und über eine Stirnzahnung 16a der Zahnstange 16 ein erstes Übertragungsritzel 19 eines ersten Doppelrades 18 aus dem Übertragungsritzel 19 und einem Kegelradkranz 15 antreibt. Ein zweiter der zwei Antriebe weist einen zweiten Motor 17' auf, der über eine Antriebswelle 17b' ein Antriebsritzel 17a' antreibt, das mit einer Seitenzahnung 16b' einer zweiten Zahnstange 16' kämmt und über eine Stirnzahnung 16a' der Zahnstange 16' ein zweites Übertragungsritzel 19' eines zweiten Doppelrades 18' aus dem Übertragungsritzel 19' und einem Kegelradkranz 15' antreibt. Dabei ist die Taumelscheibe 14 zwischen den beiden Kegelradkränzen 15,15', die einander zugewandt sind und auf einer gemeinsamen Achse A liegen, angeordnet. Ferner wird ein chirurgisches Instrument 1 mit einem solchen Lenkgetriebe 13 offenbart.

### BEZUGSZEICHENLISTE

- 1: Chirurgisches Instrument
- 2: Schaft
- 3: proximales Ende (Schaft) / Schaftendstück
- 4: Betätigungseinheit
- 5: distales Ende (Schaft)
- 6: Werkzeugspitze
- 7: Instrument
- 8: Betätigungselement
- 9: Abwinkelungsmechanik
- 10: Antriebseinheit (Taumelscheibe)
- 11: Schwenkglied
- 12: Lenkdraht
- 13: Lenkgetriebe
- 14: Taumelscheibe
- 15, 15': Kegelradkranz des jew. Doppelrades 18,18'
- 16, 16': Zahnstange
- 16a, 16a': Stirnzahnung
- 16b, 16b': seitliche Zahnung
- 16c: Schiene der Zahnstange 16
- 16d: Stirnseite
- 16e: Längsseite
- 16f: Führungsabschnitt
- 17, 17': Motor
- 17a, 17a': Antriebsritzel des jew. Doppelrades 18, 18'
- 17b, 17b': Antriebswelle
- 18, 18': Doppelrad
- 18a: Lageraugenbauteil
- 18b: Getriebewelle
- 19,19': Übertragungsritzel des jew. Doppelrades 18,18'
- 20a, b: Führungsnut, Kugelabschnitt
- 21: Hauptwelle
- 22: Fächerscheibe
- 23: Durchgangsbohrungen
- 24: Madenschrauben
- 25: drittes Zahnrad
- 27: Lagerstifte
- 28: Kreuzgelenkscheibe
- 29: Lagerstifte
- 30: Lenkring
- 31: viertes Zahnrad
- 32: Lagerring
- 33: Führungsblock für Schiene 16c der Zahnstange 16
- 33a: Führungsnut Führungsblock
- 33b: Befestigungsmittel Führungsblock
- 33c: Führungsnut in Lagerungsplatte 34
- 34: Lagerungsplatte
- 35: Durchtrittschlitz
- 36: Lagerring

- A: Mittelachse der angetriebenen Doppelzahnräder 18,18
- B: Längsachse des Instruments bzw. des Schafts
- C: Antriebsachse des ersten Antriebs 17
- C': Antriebsachse des zweiten Antriebs 17'
- D: Drehachse drittes und viertes Zahnrad

## Patentansprüche

1. Lenkgetriebe (13) für ein chirurgisches Instrument (1), wobei das Lenkgetriebe (13) zwei motorisierte Antriebe aufweist und dazu ausgebildet ist, über die Stellwinkel der zwei Antriebe eine Taumelscheibe (14) räumlich auszurichten, die dazu ausgebildet ist, eine distale Abwinkelungsmechanik (9) des chirurgischen Instruments (1) zu steuern,
wobei
ein erster der zwei Antriebe einen ersten Motor (17) aufweist, der über eine Antriebswelle (17b) ein Antriebsritzel (17a) antreibt, das mit einer Seitenzahnung (16b) einer ersten Zahnstange (16) kämmt,
und
ein zweiter der zwei Antriebe einen zweiten Motor (17') aufweist, der über eine Antriebswelle (17b') ein Antriebsritzel (17a') antreibt, das mit einer Seitenzahnung (16b') einer zweiten Zahnstange (16') kämmt, **dadurch gekennzeichnet, dass** das Antriebsritzel (17a) über eine Stirnzahnung (16a) der Zahnstange (16) ein erstes Übertragungsritzel (19) eines ersten Doppelrades (18) aus dem Übertragungsritzel (19) und einem Kegelradkranz (15) antreibt und das Antriebsritzel (17a') über eine Stirnzahnung (16a') der Zahnstange (16') ein zweites Übertragungsritzel (19') eines zweiten Doppelrades (18') aus dem Übertragungsritzel (19') und einem Kegelradkranz (15') antreibt,
und wobei die Taumelscheibe (14) zwischen den beiden Kegelradkränzen (15,15'), die einander zugewandt sind und auf einer gemeinsamen Achse (A) liegen, angeordnet ist.

2. Lenkgetriebe (13) nach Anspruch 1,
**dadurch gekennzeichnet, dass**
eine Orientierung der Antriebsachsen (C, C') unabhängig voneinander und in Bezug auf eine durch die Längsachse (B) und die gemeinsamen Achse (A) definierte Ebene festlegbar ist, wobei eine Verzahnung des Antriebsritzels (17a, 17a') mit der Seitenzahnung (16b, 16b') in Abhängigkeit der Orientierung der jeweiligen Antriebsachse (C, C') ausgebildet ist,
wobei bevorzugt eine Antriebsachse (C) des ersten Motors (17) parallel zu einer Antriebsachse (C') des zweiten Motors (17') vorliegt, wobei die Antriebsachsen (C, C') senkrecht zu der durch die Längsachse (B) und die gemeinsamen Achse (A) definierten Ebene verlaufen, und wobei bevorzugt das Antriebsritzel (17a, 17a') mit der Seitenzahnung (16b, 16b') eine Geradverzahnung bildet.

3. Lenkgetriebe (13) nach Anspruch 1 oder 2,
**dadurch gekennzeichnet, dass**
die Taumelscheibe (14) mit einem dritten Zahnrad (25) gekoppelt ist, das mit den beiden Kegelradkränzen (15,15') der beiden Doppelräder (18,18') in Eingriff steht und dessen Drehachse (D) in einem rechten Winkel zu der gemeinsamen Drehachse (A) der angetriebenen Doppelräder (18,18') steht.

4. Lenkgetriebe (13) nach zumindest einem der Ansprüche 1 bis 3,
**dadurch gekennzeichnet, dass**
die Taumelscheibe (14) mit einem vierten Zahnrad (31) gekoppelt ist, das mit den beiden Kegelradkränzen (15,15') der beiden Doppelräder (18,18') gekoppelt und auf der Seite der Taumelscheibe (14), die von dem dritten Zahnrad (25) abgewandt ist, angeordnet ist.

5. Lenkgetriebe (13) nach zumindest einem der Ansprüche 1 bis 4,
**dadurch gekennzeichnet, dass**
die Zahnstange (16, 16')
- flächig und länglich quaderförmig ist, wobei die Stirnzahnung (16a, 16a') entlang einer ersten Längsstirnseite vorliegt, und
- senkrecht zu der Stirnzahnung (16a, 16a') entlang einer Seitenfläche (16e) die Seitenzahnung (16b, 16b') aufweist, die mit dem jeweiligen Antriebsritzel (17a, 17a') in Eingriff steht.

6. Lenkgetriebe (13) nach zumindest einem der Ansprüche 1 bis 5,
**dadurch gekennzeichnet, dass**
das Lenkgetriebe (13) eine Lagerungsplatte (34) mit je einer Führungsvorrichtung für jede Zahnstange (16, 16') aufweist, wobei jede Zahnstange (16, 16') senkrecht zu der gemeinsamen Achse (A) längsbeweglich in der ihr zugeordneten Führungsvorrichtung gelagert ist und mit ihrer Stirnzahnung (16a, 16a') von einer Oberseite der Lagerungsplatte (34), auf der die Doppelräder (18, 18') mit den Übertragungsritzeln (19, 19') angeordnet sind, weg weist, wobei die Motoren (17, 17') an einer Unterseite der Lagerungsplatte (34) angeordnet sind.

7. Lenkgetriebe (13) nach Anspruch 6,
**dadurch gekennzeichnet, dass**
die Führungsvorrichtungen
- jeweils eine senkrecht zu der gemeinsamen Achse (A) verlaufende Führungsnut (33c, 33c') in der Lagerungsplatte (34) sind, wobei ein unterer Führungsabschnitt (16f) an der von der Stirnzahnung (16a, 16a') abgewandten Seite der Zahnstange (16, 16')in der Führungsnut (33b, 33b') aufgenommen ist,
und/oder
- durch zumindest einen an der Oberseite der Lagerungsplatte (34), angeordneten Führungsblock (33, 33') mit einer senkrecht zu der gemeinsamen Achse (A) verlaufenden und zu der jeweiligen Zahnstange (16,16') weisenden seitlichen Führungsnut (33a, 33a')bereitgestellt werden, in der eine Längsschiene (16c) aufgenommen ist, die an einer von der Seitenfläche (16e) mit der Seitenzahnung (16b, 16b') abgewandten Seitenfläche der Zahnstange (16, 16') vorliegt.

8. Lenkgetriebe (13) nach Anspruch 6 oder 7,
**dadurch gekennzeichnet, dass**
die Seitenfläche (16e) der Zahnstangen (16, 16') mit der Seitenzahnung (16b, 16b') dem jeweiligen Doppelrad (18, 18') zugewandt ist.

9. Lenkgetriebe (13) nach zumindest einem der Ansprüche 1 bis 5,
**dadurch gekennzeichnet, dass**
jedes Doppelrad (18,18') ein Kegelrad mit dem Kegelradkranz (15, 15') aufweist, das mit dem Übertragungsritzel (19, 19'), das eine umlaufende Zahnung aufweist, über eine Getriebewelle (18b) verbunden ist, die sich entlang der gemeinsamen Achse (A) erstreckt.

10. Lenkgetriebe (13) nach Anspruch 9,
**dadurch gekennzeichnet, dass**
die Getriebewelle (18b) in einem Lageraugenbauteil (18a, 18a') drehbar gelagert ist, wobei das Lageraugenbauteil (18a, 18a') auf der Lagerungsplatte (34) befestigt ist und in Bezug auf die Drehachse (A) des mit der Getriebewelle (18b) in dem Lageraugenbauteil (18a, 18a') gelagerten Doppelrades (18, 18') ausgerichtet ist.

11. Chirurgisches Instrument (1), das einen Schaft (2), eine am proximalen Ende (3) des Schaftes (2) angeordnete Betätigungseinheit (4) und ein am distalen Ende (5) des Schaftes (2) angeordnetes Instrument (7) mit einer mittels einer distalen Abwinkelungsmechanik (9) abwinkelbaren Werkzeugspitze (6), die durch eine mittels zweier Antriebe räumlich ausrichtbare Taumelscheibe (14) steuerbar ist, **dadurch gekennzeichnet, dass**
das chirurgische Instrument (1) ein Lenkgetriebe (13) nach zumindest einem der Ansprüche 1 bis 10 aufweist, das die zwei Antriebe aufweist und dazu ausgebildet ist, die Stellwinkel der zwei Antriebe auf die räumliche Ausrichtung der Taumelscheibe (14) zu übertragen.

12. Chirurgisches Instrument (1) nach Anspruch 11,
**dadurch gekennzeichnet, dass**
die Taumelscheibe (14) zur Kopplung mit einem dritten Zahnrad (25), das mit den beiden Kegelradkränzen (15, 15') der beiden Doppelräder (18, 18') in Eingriff steht, um die Längsachse (B) des Schaftes (2) über einen Lagerring (32) rotierbar in einem Lenkring (30) gelagert ist, der drehfest mit dem dritten Zahnrad (25) gekoppelt ist, wobei die Taumelscheibe (14) kardanisch mit einer koaxial zu einer Längsachse (B) des Schaftes (2) verlaufenden Hauptwelle (21) gekoppelt ist.

13. Chirurgisches Instrument (1) nach Anspruch 11 oder 12,
**dadurch gekennzeichnet, dass**
die Taumelscheibe (14) über zwei um 180 ° versetzt zueinander angeordnete Lagerstifte (27) verschwenkbar auf einer Kreuzgelenkscheibe (28) gelagert ist, wobei die Kreuzgelenkscheibe (28) über zwei um 180° versetzt zueinander angeordnete Lagerstifte (29) verschwenkbar auf der Hauptwelle (21) gelagert ist und wobei die Lagerstifte (27, 29) der Taumelscheibe (14) und der Kreuzgelenkscheibe (28) um 90 ° versetzt zueinander angeordnet sind,
oder die kardanische Lagerung durch zwei sich längs erstreckende, diametral in der Hauptwelle (21) vorliegende Führungsnuten (20a) und zwei diametral und radial nach innen weisend an der Taumelscheibe (14) angeordnete Stifte (29) bereitgestellt wird, wobei jeder Stift (29) in eine der Führungsnuten (20a) eingreift, sodass ein Drehwinkel der Hauptwelle (21) auf die Taumelscheibe (14) übertragbar ist.

14. Chirurgisches Instrument (1) nach zumindest einem der Ansprüche 11 bis 13, **dadurch gekennzeichnet, dass**
das vierte Zahnrad (31) mit der Taumelscheibe (14) über einen Lagerring (42) mit dem Lenkring (30) gekoppelt ist, wobei das vierte Zahnrad (31) gegenüber dem dritten Zahnrad (25) frei drehbar ist.

15. Chirurgisches Instrument (1) nach zumindest einem der Ansprüche 11 bis 14, **dadurch gekennzeichnet, dass**
ein Betätigungselement (8) axial verschiebbar in dem Schaft (2) gelagert ist und proximalseitig mit der Betätigungseinheit (4) in Wirkverbindung steht, und dass die distalen Abwinkelungsmechanik (9) der abwinkelbaren Werkzeugspitze (6) aus an dem distalen Ende (5) des Schaftes (2) angeordneten Schwenkgliedern (11) besteht, die über in Längsrichtung des Schaftes (2) verlaufende Lenkdrähte (12) mit dem Lenkgetriebe (13) verbunden sind.

## Claims

1. A steering gear (13) for a surgical instrument (1), wherein the steering gear (13) has two motorised drives and is designed to spatially align a swash plate (14) via the adjustment angles of the two drives, which swash plate is designed to control a distal bending mechanism (9) of the surgical instrument (1),
wherein
a first of the two drives has a first motor (17) which drives a drive pinion (17a) via a drive shaft (17b), which drive pinion meshes with a lateral toothing (16b) of a first toothed rack (16),
and
a second of the two drives has a second motor (17') which drives a drive pinion (17a') via a drive shaft (17b'), which drive pinion meshes with a lateral toothing (16b') of a second toothed rack (16'), **characterised in that** the drive pinion (17a) drives a first transmission pinion (19) of a first double wheel (18) consisting of the transmission pinion (19) and a bevel gear rim (15) via an end toothing (16a) of the toothed rack (16), and the drive pinion (17a') drives a second transmission pinion (19') of a second double wheel (18') consisting of the transmission pinion (19') and a bevel gear rim (15') via an end toothing (16a') of the toothed rack (16'), and wherein the swash plate (14) is arranged between the two bevel gear rims (15, 15'), which face each other and lie on a common axis (A).

2. The steering gear (13) according to claim 1,
**characterised in that**
an orientation of the drive axes (C, C') can be fixed independently of one another and in relation to a plane defined by the longitudinal axis (B) and the common axis (A), wherein a toothing of the drive pinion (17a, 17a') with the lateral toothing (16b, 16b') is formed as a function of the orientation of the respective drive axis (C, C'),
wherein preferably a drive axis (C) of the first motor (17) is parallel to a drive axis (C') of the second motor (17'), wherein the drive axes (C, C') extend perpendicular to the plane defined by the longitudinal axis (B) and the common axis (A), and wherein preferably the drive pinion (17a, 17a') forms a spur toothing with the lateral toothing (16b, 16b').

3. The steering gear (13) according to claim 1 or 2,
**characterised in that**
the swash plate (14) is coupled to a third gear wheel (25), which is in engagement with the two bevel gear rims (15, 15') of the two double wheels (18, 18') and whose axis of rotation (D) is at a right angle to the common axis of rotation (A) of the driven double wheels (18, 18').

4. The steering gear (13) according to at least one of claims 1 to 3, **characterised in that**
the swash plate (14) is coupled to a fourth gear wheel (31), which is coupled to the two bevel gear rims (15, 15') of the two double wheels (18, 18') and is arranged on the side of the swash plate (14) facing away from the third gear wheel (25).

5. The steering gear (13) according to at least one of claims 1 to 4, **characterised in that**
the toothed rack (16, 16')
- is planar and elongated in a cuboid shape, wherein the end toothing (16a, 16a') is present along a first longitudinal end face, and
- has, perpendicular to the end toothing (16a, 16a') along a side surface (16e), the lateral toothing (16b, 16b'), which is in engagement with the respective drive pinion (17a, 17a').

6. The steering gear (13) according to at least one of claims 1 to 5, **characterised in that**
the steering gear (13) has a bearing plate (34) with a guide device for each toothed rack (16, 16'), wherein each toothed rack (16, 16') is mounted perpendicular to the common axis (A) so as to be longitudinally movable in the guide device assigned thereto and faces with its end toothing (16a, 16a') away from an upper side of the bearing plate (34) on which are arranged the double wheels (18, 18') with the transmission pinions (19, 19'), wherein the motors (17, 17') are arranged on an underside of the bearing plate (34).

7. The steering gear (13) according to claim 6,
**characterised in that**
the guide devices
- are each a guide groove (33c, 33c') running perpendicular to the common axis (A) in the bearing plate (34), wherein a lower guide section (16f) is received in the guide groove (33b, 33b') on the side of the toothed rack (16, 16') facing away from the end toothing (16a, 16a'),
and/or
- are provided by at least one guide block (33, 33') arranged on the upper side of the bearing plate (34), with a lateral guide groove (33a, 33a') running perpendicular to the common axis (A) and pointing towards the respective toothed rack (16, 16'), in which guide groove a longitudinal rail (16c) is received, which is present on a side surface of the toothed rack (16, 16') facing away from the side surface (16e) with the lateral toothing (16b, 16b').

8. The steering gear (13) according to claim 6 or 7,
**characterised in that**
the side surface (16e) of the toothed racks (16, 16') with the lateral toothing (16b, 16b') faces the respective double wheel (18, 18').

9. The steering gear (13) according to at least one of claims 1 to 5, **characterised in that**
each double wheel (18, 18') has a bevel gear with the bevel gear rim (15, 15'), which is connected to the transmission pinion (19, 19'), which has a circumferential toothing, via a transmission shaft (18b) extending along the common axis (A).

10. The steering gear (13) according to claim 9,
**characterised in that**
the transmission shaft (18b) is rotatably mounted in a bearing eye component (18a, 18a'), wherein the bearing eye component (18a, 18a') is fastened on the bearing plate (34) and is aligned in relation to the axis of rotation (A) of the double wheel (18, 18') mounted with the transmission shaft (18b) in the bearing eye component (18a, 18a').

11. A surgical instrument (1) comprising a shaft (2), an actuation unit (4) arranged at the proximal end (3) of the shaft (2) and an instrument (7) arranged at the distal end (5) of the shaft (2) with a tool tip (6) which can be angled by means of a distal bending mechanism (9) and which can be controlled by a swash plate (14) which can be spatially aligned by means of two drives,
**characterised in that**
the surgical instrument (1) has a steering gear (13) according to at least one of claims 1 to 10, which has the two drives and is designed to transmit the adjustment angles of the two drives to the spatial orientation of the swash plate (14).

12. The surgical instrument (1) according to claim 11,
**characterised in that**
the swash plate (14), for coupling with a third gear wheel (25), which is in engagement with the two bevel gear rims (15, 15') of the two double wheels (18, 18'), is mounted rotatably about the longitudinal axis (B) of the shaft (2) via a bearing ring (32) in a steering ring (30), which is coupled to the third gear wheel (25) in a rotationally-fixed manner, wherein the swash plate (14) is coupled in a cardanic manner to a main shaft (21) running coaxially to a longitudinal axis (B) of the shaft (2).

13. The surgical instrument (1) according to claim 11 or 12,
**characterised in that**
the swash plate (14) is pivotably mounted on a universal joint disc (28) via two bearing pins (27) arranged offset by 180° relative to one another, wherein the universal joint disc (28) is pivotably mounted on the main shaft (21) via two bearing pins (29) arranged offset by 180° relative to one another and wherein the bearing pins (27, 29) of the swash plate (14) and the universal joint disc (28) are arranged offset by 90° relative to one another,
or the cardanic mounting is provided by two longitudinally extending guide grooves (20a) located diametrically in the main shaft (21) and two pins (29) arranged diametrically and pointing radially inwards on the swash plate (14), wherein each pin (29) engages into one of the guide grooves (20a) such that an angle of rotation of the main shaft (21) can be transmitted to the swash plate (14).

14. The surgical instrument (1) according to at least one of claims 11 to 13, **characterised in that**
the fourth gear wheel (31) is coupled to the swash plate (14) via a bearing ring (42) with the steering ring (30), wherein the fourth gear wheel (31) is freely rotatable relative to the third gear wheel (25).

15. The surgical instrument (1) according to at least one of claims 11 to 14, **characterised in that**
an actuating element (8) is mounted so as to be axially displaceable in the shaft (2) and is operatively connected to the actuation unit (4) proximally at the side, and **in that** the distal bending mechanism (9) of the bendable tool tip (6) consists of swivel members (11) which are arranged at the distal end (5) of the shaft (2) and which are connected to the steering gear (13) via steering wires (12) running in the longitudinal direction of the shaft (2).

## Revendications

1. Appareil de direction (13) pour un instrument chirurgical (1), dans lequel le mécanisme de direction (13) a deux entraînements motorisés et étant conçu de manière à orienter dans l'espace un plateau oscillant (14) par l'intermédiaire des angles de réglage des deux entraînements, ce plateau oscillant étant conçu de manière à commander un mécanisme de flexion distal (9) de l'instrument chirurgical (1),
dans lequel
le premier des deux entraînements a un premier moteur (17) qui entraîne un pignon d'entraînement (17a) par l'intermédiaire d'un arbre d'entraînement (17b), ce pignon d'entraînement s'engrène avec une denture latérale (16b) d'une première crémaillère (16),
et
le second des deux entraînements a un second moteur (17') qui entraîne un pignon d'entraînement (17a') par l'intermédiaire d'un arbre d'entraînement (17b'), ce pignon d'entraînement s'engrène avec une denture latérale (16b') d'une seconde crémaillère (16'), **caractérisé en ce que** le pignon d'entraînement (17a) entraîne un premier pignon de transmission (19) d'une première roue double (18) constituée du pignon de transmission (19) et d'une couronne conique (15) par l'intermédiaire d'une denture d'extrémité (16a) de la crémaillère (16), et le pignon d'entraînement (17a') entraîne un second pignon de transmission (19') d'une seconde roue double (18') constituée du pignon de transmission (19') et d'une couronne conique (15') par l'intermédiaire d'une denture d'extrémité (16a') de la crémaillère (16'),
et dans lequel le plateau oscillant (14) est disposé entre les deux couronnes coniques (15, 15'), qui se font face et se trouvent sur un axe commun (A).

2. Appareil de direction (13) selon la revendication 1,
**caractérisé en ce que**
une orientation des axes d'entraînement (C, C') peut être fixée indépendamment les uns des autres et par rapport à un plan défini par l'axe longitudinal (B) et l'axe commun (A), dans lequel une denture du pignon d'entraînement (17a, 17a') avec la denture latérale (16b, 16b') est formée en fonction de l'orientation de l'axe d'entraînement respectif (C, C'),
dans lequel, de préférence, un axe d'entraînement (C) du premier moteur (17) est parallèle à un axe d'entraînement (C') du second moteur (17'), dans lequel les axes d'entraînement (C, C') s'étendent perpendiculairement au plan défini par l'axe longitudinal (B) et l'axe commun (A), et dans lequel, de préférence, le pignon d'entraînement (17a, 17a') forme une denture droite avec la denture latérale (16b, 16b').

3. Appareil de direction (13) selon la revendication 1 ou 2,
**caractérisé en ce que**
le plateau oscillant (14) est couplé à une troisième roue dentée (25), qui est en prise avec les deux couronnes coniques (15, 15') des deux roues doubles (18, 18') et dont l'axe de rotation (D) est à angle droit par rapport à l'axe de rotation commun (A) des roues doubles entraînées (18, 18').

4. Appareil de direction (13) selon au moins l'une des revendications 1 à 3, **caractérisé en ce que**
le plateau oscillant (14) est couplé à une quatrième roue dentée (31), qui est couplée aux deux couronnes coniques (15, 15') des deux roues doubles (18, 18') et qui est disposée sur le côté du plateau oscillant (14) opposé à la troisième roue dentée (25).

5. Appareil de direction (13) selon au moins l'une des revendications 1 à 4, **caractérisé en ce que**
la crémaillère (16, 16')
- est plane et allongée en forme de parallélépipède, dans lequel la denture d'extrémité (16a, 16a') est présente le long d'une première face d'extrémité longitudinale, et
- a, perpendiculaire à la denture d'extrémité (16a, 16a') le long d'une surface latérale (16e), la denture latérale (16b, 16b'), qui est en prise avec le pignon d'entraînement (17a, 17a') respectif.

6. Appareil de direction (13) selon au moins l'une des revendications 1 à 5, **caractérisé en ce que**
l'appareil de direction (13) a une plaque d'appui (34) avec un dispositif de guidage pour chaque crémaillère (16, 16'), dans lequel chaque crémaillère (16, 16') est montée perpendiculairement à l'axe commun (A) de manière à pouvoir se déplacer longitudinalement dans le dispositif de guidage qui lui est affecté et à faire face à sa denture d'extrémité (16a, 16a') loin d'un côté supérieur de la plaque d'appui (34) sur laquelle sont disposées les roues doubles (18, 18') avec les pignons de transmission (19, 19'), dans lequel les moteurs (17, 17') sont disposés sur une face inférieure de la plaque d'appui (34).

7. Appareil de direction (13) selon la revendication 6,
**caractérisé en ce que**
les dispositifs de guidage
- sont chacune une rainure de guidage (33c, 33c') perpendiculaire à l'axe commun (A) de la plaque d'appui (34), dans lequel une section de guidage inférieure (16f) est reçue dans la rainure de guidage (33b, 33b') sur le côté de la crémaillère (16, 16') opposé à la denture d'extrémité (16a, 16a'),
et/ou
- sont fournis par au moins un bloc de guidage (33, 33') disposé sur la face supérieure de la plaque d'appui (34), avec une rainure de guidage latérale (33a, 33a') perpendiculaire à l'axe commun (A) et orientée vers les crémaillères respectives (16, 16'), cette rainure de guidage recevant un rail longitudinal (16c), qui est présente sur une surface latérale de la crémaillère (16, 16') opposée à la surface latérale (16e) avec la denture latérale (16b, 16b').

8. Appareil de direction (13) selon la revendication 6 ou 7,
**caractérisé en ce que**
la surface latérale (16e) des crémaillères (16, 16') avec la denture latérale (16b), 16b') fait face à la roue double (18, 18') respective.

9. Appareil de direction (13) selon au moins l'une des revendications 1 à 5, **caractérisé en ce que**
chaque roue double (18, 18') a un engrenage conique avec la couronne conique (15, 15'), qui est relié au pignon de transmission (19, 19'), qui a une denture circonférentielle, par l'intermédiaire d'un arbre de transmission (18b) s'étendant le long de l'axe commun (A).

10. Appareil de direction (13) selon la revendication 9,
**caractérisé en ce que**
l'arbre de transmission (18b) est monté rotatif dans un élément d'oeillet de palier (18a), 18a'), dans lequel le élément d'oeillet de palier (18a, 18a') est fixé sur la plaque d'appui (34) et est aligné par rapport à l'axe de rotation (A) de la roue double (18, 18') montée avec l'arbre de transmission (18b) dans l'élément d'oeillet de palier (18a, 18a').

11. Instrument chirurgical (1) comprenant une tige (2), une unité d'actionnement (4) disposée à l'extrémité proximale (3) de l'arbre (2) et un instrument (7) disposé à l'extrémité distale (5) de l'arbre (2) avec une pointe d'outil (6) qui peut être inclinée au moyen d'un mécanisme de flexion distale (9) et qui peut être contrôlée par un plateau oscillant (14) qui peut être aligné dans l'espace au moyen de deux entraînements,
**caractérisé en ce que**
l'instrument chirurgical (1) a un appareil de direction (13) selon au moins l'une des revendications 1 à 10, qui a les deux entraînements et qui est conçu pour transmettre les angles de réglage des deux entraînements à l'orientation spatiale du plateau oscillant (14).

12. Instrument chirurgical (1) selon la revendication 11,
**caractérisé en ce que**
le plateau oscillant (14), pour l'accouplement avec une troisième roue dentée (25), qui est en prise avec les deux couronnes coniques (15, 15') des deux roues doubles (18, 18'), est monté rotatif autour de l'axe longitudinal (B) de l'arbre (2) par l'intermédiaire d'une bague de roulement (32) dans une bague de direction (30), qui est couplée à la troisième roue dentée (25) de manière fixe en rotation, dans lequel le plateau oscillant (14) est couplé de manière cardanique à un arbre principal (21) coaxial à l'axe longitudinal (B) de l'arbre (2).

13. Instrument chirurgical (1) selon la revendication 11 ou 12,
**caractérisé en ce que**
le plateau oscillant (14) est monté pivotant sur un disque de joint universel (28) par l'intermédiaire de deux goupilles d'appui (27) décalés de 180° l'un par rapport à l'autre, dans lequel le disque de joint universel (28) est monté pivotant sur l'arbre principal (21) par l'intermédiaire de deux goupilles d'appui (29) décalés de 180° l'un par rapport à l'autre et dans lequel les goupilles d'appui (27, 29) du plateau oscillant (14) et du disque de joint universel (28) sont décalés de 90° l'un par rapport à l'autre,
ou le montage cardanique est fourni par deux rainures de guidage (20a) s'étendant longitudinalement et situées diamétralement dans l'arbre principal (21) et deux goupilles (29) disposées diamétralement et orientées radialement vers l'intérieur sur le plateau oscillant (14), dans lequel chaque goupille (29) s'engage dans l'une des rainures de guidage (20a) de sorte qu'un angle de rotation de l'arbre principal (21) puisse être transmis au plateau oscillant (14).

14. Instrument chirurgical (1) selon au moins l'une des revendications 11 à 13, **caractérisé en ce que**
la quatrième roue dentée (31) est couplée au plateau oscillant (14) par l'intermédiaire d'une bague de roulement (42) avec la bague de direction (30), dans lequel la quatrième roue dentée (31) peut tourner librement par rapport à la troisième roue dentée (25).

15. Instrument chirurgical (1) selon au moins l'une des revendications 11 à 14, **caractérisé en ce que**
un élément d'actionnement (8) est monté de manière à pouvoir être déplacé axialement dans l'arbre (2) et est relié de manière opérationnelle à l'unité d'actionnement (4) proximalement sur le côté, et **en ce que** le mécanisme de flexion distale (9) de la pointe de l'outil pliable (6) constitue des éléments pivotants (11) qui sont disposés à l'extrémité distale (5) de l'arbre (2) et qui sont reliés à l'appareil de direction (13) par des fils de direction (12) s'étendant dans la direction longitudinale de l'arbre (2).
